# EUROPEAN PATENT APPLICATION

(11) **EP 3 396 674 A1**
(43) Date of publication of application: **31.10.2018**
(21) Application number: 18160252.5
(22) Date of filing: 06.03.2018
(51) Int. Cl.: G16H 20/40, A61H 1/00

(54) **MASSAGE APPARATUS AND MASSAGE SYSTEM**

(30) Priority: 27.04.2017 JP 2017087906
(71) Applicant: Family Inada Co., Ltd., Osaka, Osaka 532-0004 (JP)
(72) Inventor: INADA, Nichimu, Osaka-shi, Osaka 532-0004 (JP); ISHIDOU, Yuta, Saihaku-gun, Tottori 689-3224 (JP)
(74) Representative: Horn Kleimann Waitzhofer Patentanwälte PartG mbB

(57) **Abstract**

The invention provides a massage apparatus capable of obtaining at least either diagnosis in accordance with symptoms of a user or appropriate advice by using artificial intelligence. Provided is a massage apparatus that has a massage section for massaging a user, the apparatus including: a sound input section; a sound output section; and an artificial intelligence section that determines diagnosis and advice based on data accumulated at least in relation to knowledge and know-how of doctors, in which the artificial intelligence section is configured to determine at least either of diagnosis in accordance with the user or appropriate advice based on the data at least in relation to the knowledge and the know-how of the doctors, on the basis of sound information of the user input from the sound input section through a conversation with the user and outputs at least either the diagnosis or the advice from the sound output section.

## Description

### TECHNICAL FIELD

The present invention relates to a massage apparatus and a massage system that massage each part of a user.

### BACKGROUND ART

In the related art, there is a massage apparatus that massages each part of a body of a user. For example, there are a handy massage apparatus that massages shoulders and a neck, a cushion massage apparatus that massages a lower back and a back, a foot massage apparatus that massages legs and carves, a chair-type massage apparatus that massages a lower back, a back, and shoulders and the like. Users use such massage apparatuses about once a day, for example.

The users who use the massage apparatuses use the massage apparatuses for alleviating symptoms, such as shoulder stiffness, lower back pain, and leg fatigue, at that time. Thus, there is a massage machine that conducts medical interview for a user and determines a physical condition of the user on the basis of answers from the user in order to perform appropriate massage in accordance with the physical condition of the user (see Patent Document 1, for example). According to this massage machine, the medical interview is conducted for the user, and the user answers the medical interview with either "Yes" or "No".

### RELATED ART DOCUMENT

### PATENT DOCUMENT

Patent Document 1]: JP-A-2000-70318

### SUMMARY OF THE INVENTION

### PROBLEM THAT THE INVENTION IS TO SOLVE

Incidentally, a user who uses a massage apparatus may desire to know reasons and countermeasures for symptoms of a poor physical condition or the like, if any. In such a case, it is necessary for the user to visit a hospital that matches the symptoms, to get diagnosis by a medical specialist of the symptoms, and ask for reasons, a disease name, appropriate advice for the symptoms, and the like in order to know the reasons for the symptoms of the poor physical condition or the like. However, although there are many doctors who have thorough knowledge about each specialized field of medicines, there are not many doctors who have thorough knowledge about the entire field of medicines and all symptoms of affected parts. Therefore, there is a case in which it is not possible to know what disease or symptom the user themselves has suffered from as long as the user cannot be checked by a medical specialist of a corresponding field. Further, there is also a case in which a healthcare facility is located far away or a case of bad weather. Therefore, the user may hesitate to go to the healthcare facility even if there are symptoms of a poor physical condition or the like.

In addition, the user may desire to know an appropriate massage course or the like for the physical condition of their own in a case in which there are symptoms of a poor physical condition or the like.

Note that since the content of the medical interview conducted for the user by the massage machine in Patent Document 1 is content set in advance, and the user responds to the medical interview with either "Yes" or "No", the content of massage selected on the basis of the determination of the physical condition is limited. Further, it is not possible to provide appropriate advice for the poor physical condition or the like of the user.

Thus, an object of the present invention is to provide a massage apparatus and a massage system capable of obtaining at least either diagnosis in accordance with symptoms of the user or appropriate advice by utilizing artificial intelligence.

### MEANS FOR SOLVING THE PROBLEM

In order to achieve the aforementioned object, a massage apparatus according to the present invention is a massage apparatus that has a massage section for massaging a user, the apparatus including: a sound input section; a sound output section; and an artificial intelligence section that determines diagnosis and advice based on data accumulated at least in relation to knowledge and know-how of doctors, in which the artificial intelligence section is configured to determine at least either of diagnosis in accordance with the user or appropriate advice based on the data at least in relation to the knowledge and the know-how of the doctors, on the basis of sound information of the user input from the sound input section through a conversation with the user and outputs at least either the diagnosis or the advice from the sound output section. The "sound information of the user" in this specification and the claims include information related to a physical condition, information related to symptoms, and information related to massage. Knowledge of various fields can be accumulated in addition to the knowledge and know-how of the doctors as the data accumulated in the artificial intelligence section.

With this configuration, the artificial intelligence section determines the diagnosis or the advice in accordance with the user on the basis of the data accumulated in relation to the knowledge and the know-how of the doctors and outputs the diagnosis or the advice from the sound output section if the user inputs the sound information including the physical condition or the like from the sound input section of the massage apparatus. In this manner, the user can obtain the diagnosis in accordance with the symptoms of the user themselves or the appropriate advice through a conversation with the massage apparatus. Since the user can obtain the diagnosis or the appropriate advice in relation to the physical condition or the like from the massage apparatus, it is possible to reduce a time, cost, and an effort to go to a hospital.

In addition, the artificial intelligence section may be configured to determine at least either the diagnosis in accordance with the user or the appropriate advice based on the data accumulated at least in relation to knowledge and know-how of a plurality of doctors in a plurality of different fields. The "different fields" in this specification and the claims include different fields of hospital departments, different fields of specialties in the same hospital department, and the like.

With this configuration, the artificial intelligence section can determine the diagnosis in accordance with the user or the appropriate advice from diversified standpoints on the basis of the knowledge and the know-how of the plurality of doctors from different hospital departments or different specialized fields in response to sound information of the user.

In addition, the massage apparatus may further include an item selection section through which the user provides inputs, and the artificial intelligence section may be configured to determine at least either the diagnosis in accordance with the user or the appropriate advice based on the data accumulated in relation to the knowledge and know-how of the doctors, on the basis of the sound information of the user, in relation to an item selected by the user through the item selection section. The "item selection section" in this specification and the claims includes items such as a "fever", a "blood pressure", "shoulder stiffness", "lower back pain", a "headache", a "stomachache", "constipation", "dizziness", "swelling", "urinary symptoms", "insomnia", "depressive symptoms", and the like.

With this configuration, it is possible to further improve a time and accuracy of the diagnosis and the appropriate advice provided by the artificial intelligence section by the user selecting symptoms of interest through the item selection section.

In addition, the massage apparatus may include a body state acquisition section that acquires a body state of the user, and the artificial intelligence section may be configured to determine at least either the diagnosis in accordance with the user or the appropriate advice in consideration of the body state obtained by the body state acquisition section in addition to the sound information of the user. The "body state acquisition section" in this specification and the claims include a blood pressure monitor, a body composition analyzer, a stress index measuring instrument, and the like. The "body state" includes a "pulse rate", a "body weight", a "body fat percentage", a "stress index", and the like.

With this configuration, the artificial intelligence section can provide more detailed diagnosis or appropriate advice to the user on the basis of the sound information and the body state of the user.

In addition, the massage apparatus may include a storage section that stores individual information of the user, and the artificial intelligence section may be configured to determine at least either the diagnosis in accordance with the user or the appropriate advice in consideration of the individual information saved in the storage section in addition to the sound information of the user. The "individual information" in this specification and the claims can include an "age", a "body weight", a "height", a "sex", a "family configuration", a "family medical history", and the like.

With this configuration, the artificial intelligence section can provide the diagnosis or the appropriate advice about possible diseases and countermeasures therefor to the user in consideration of the individual information in addition to the sound information of the user. For example, it is possible to letting the user to know a concern of a disease or to recommend an appropriate hospital department in accordance with the symptoms on the basis of the sound information and the family medical history of the user.

In addition, the massage apparatus may include an imaging section that images the user, and the artificial intelligence section may be configured to determine at least either the diagnosis in accordance with the user or the appropriate advice in consideration of imaging information captured by the imaging section in addition to the sound information of the user.

With this configuration, the artificial intelligence section can provide the diagnosis in accordance with the user, the appropriate advice, and advice such as suggestion of an exercise menu, suggestion of a food menu, or the like in consideration of imaging information, such as a skin color, skin firmness, and the like of the user, in addition to the sound information of the user.

In addition, the artificial intelligence section may be configured to accumulate at least either the diagnosis or the appropriate advice for the user in the information storage section and determine at least either new diagnosis in accordance with the user at the time of reuse thereof or appropriate advice in consideration of at least either the diagnosis or the appropriate advice accumulated in the information storage section in addition to sound information of the user at the time of the reuse thereof.

With this configuration, the artificial intelligence section can determine the diagnosis in accordance with the user at the time of reuse thereof and the appropriate advice by comparing and examining the information accumulated in the information storage section and the sound information of the user at the time of the reuse thereof. The artificial intelligence section can include a so-called follow-up diagnosis function. The follow-up diagnosis function enables reduction of the time, the cost, and the effort for going to a hospital again.

In addition, the massage apparatus may include a clock section that has at least date and time information, and the artificial intelligence section may be configured to determine at least either the diagnosis in accordance with the user or the appropriate advice in consideration of use date and time information of the user obtained from the clock section.

With this configuration, the artificial intelligence section can determine the diagnosis in accordance with the user and the appropriate advice in consideration of the use date and time information, such as an epidemic period of "influenza" or the like, a period with a large temperature difference due to a change in seasons, and the like, in addition to the sound information of the user.

In addition, any of the aforementioned massage apparatuses may be a chair-type massage apparatus that has at least a seat section on which the user is seated, a backrest section that supports, from a back side, an upper body of the user who is seated on the seat section, the massage section that performs massage, and a control section that controls the massage section, and the artificial intelligence section may be configured to conduct palpation with the massage section on the user who is seated on the seat section and determine at least either the diagnosis in accordance with the user and the appropriate advice in consideration of a result of the palpation in addition to the sound information of the user.

With this configuration, the artificial intelligence section can perform palpation on the user by the massage section, for example, and determine the diagnosis in accordance with the user or the appropriate advice in consideration of a result of the palpation. Muscular stiffness obtained by the palpation can be represented to the user through a mapping image or the like. It is possible to utilize a relationship between accumulated muscular stiffness and a health state for diagnosis or advice at the time of the next use by the user by accumulating the muscular stiffness of the user as data linked with the health state.

In addition, the artificial intelligence section may be configured to recommend an appropriate massage course or encourage inhibition of the massage on the basis of a result of determining at least either the diagnosis or the appropriate advice.

With this configuration, the artificial intelligence section can recommend the appropriate massage course for the user on the basis of a result of the determination of at least either the diagnosis of the user or the appropriate advice. For example, it is possible to recommend a massage course or the like in which massage is performed intensively on a specific part on the basis of the result of the determination in consideration of the body state. In a case in which it is determined that the massage had better not be performed as a result, it is possible to encourage inhibition of the massage.

Meanwhile, a massage system according to the present invention is a massage system including: any one of the aforementioned massage apparatuses, in which the artificial intelligence section is connected to a server via a communication network and is configured to be able to update the accumulated data related to the knowledge and the know-how of the doctors.

With this configuration, it is possible to update the data related to the knowledge and the know-how of the doctors to the latest state via the communication network. Therefore, it is possible for the user to obtain the diagnosis or the appropriate advice on the basis of the latest knowledge and the know-how at the time of use thereof.

### ADVANTAGE OF THE INVENTION

According to the present invention, the user who uses the massage apparatus can obtain at least either diagnosis in accordance with symptoms of the user themselves or appropriate advice through a conversation with the artificial intelligence section. The user can determine to go to a healthcare facility or the like by referring to the obtained diagnosis or advice. In addition, the user can also select a massage course appropriate for the symptoms of the user themselves.

### BRIEF DESCRIPTION OF THE DRAWINGS

- Fig. 1: is a perspective view illustrating a chair-type massage apparatus according to an embodiment of the present invention.
- Fig. 2: is a functional block diagram of the massage apparatus illustrated in Fig. 1.
- Fig. 3: is a diagram illustrating an example of a conversation between the massage apparatus illustrated in FIG. 1 and a user and advice.
- Fig. 4: is a diagram illustrating an example of a conversation between the massage apparatus illustrated in Fig 1 and the user and an example of advice, (A) is a diagram illustrating an example of item selection, and (B) is a diagram illustrating a specific example of a conversation.
- Fig. 5: is a diagram illustrating a specific example of a conversation that is different from the example of the conversation between the massage apparatus and the user illustrated in Fig. 4.
- Figs. 6(A) to (C): are diagrams illustrating palpation of the massage apparatus illustrated in Fig. 1, an example of a mapping image of muscular stiffness, and a relationship with an artificial intelligence section.
- Figs. 7(A) and (B): are diagrams illustrating an example in which data associating a mapping image of the muscular stiffness illustrated in Fig. 6 with a health state of the user are accumulated.

### BEST MODE FOR CARRYING OUT THE INVENTION

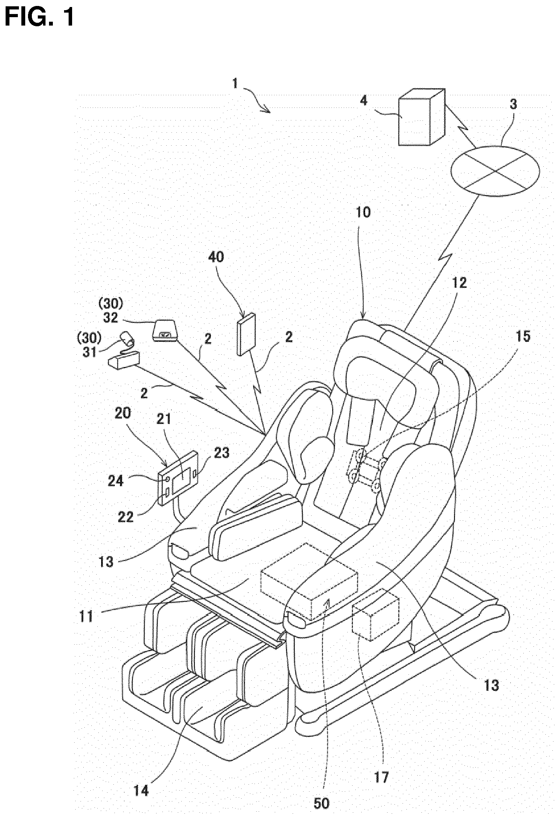

Hereinafter, an embodiment of the present invention will be described with reference to drawings. In the following embodiment, a chair-type massage apparatus 10 (hereinafter, also simply referred to as a "massage apparatus 10") will be described as an example of the massage apparatus. Concepts of the front-back direction and the left-right direction in this specification and the claims coincide with the concepts of the directions when viewed from a user when the user is seated on a seat section 11 of the massage apparatus 10 illustrated in Fig. 1.

### Configuration of chair-type massage apparatus

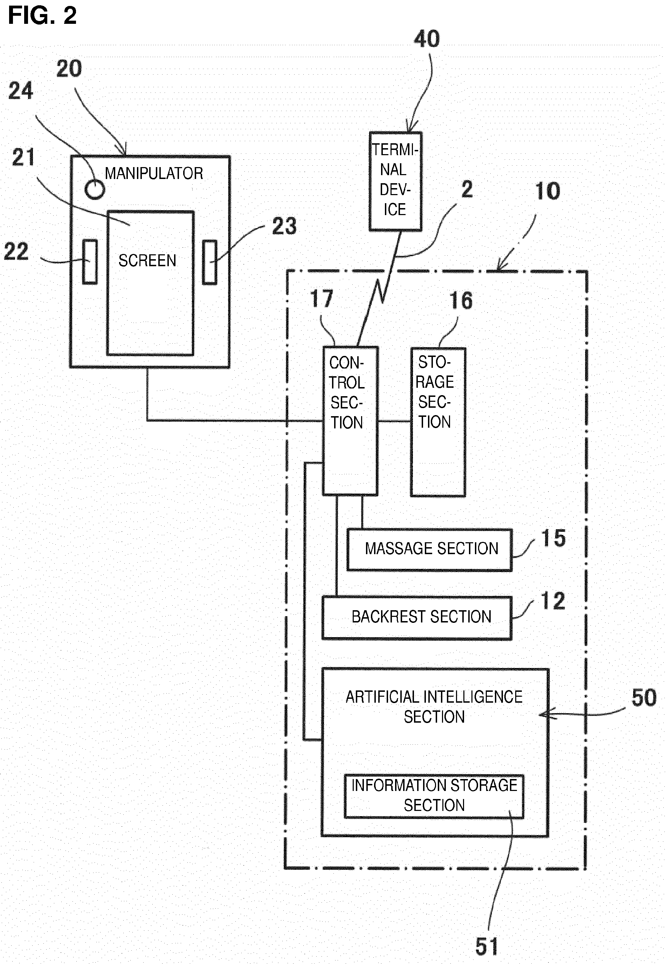

Fig. 1 is a perspective view illustrating the chair-type massage apparatus 10 according to an embodiment, and Fig. 2 is a functional block diagram of the chair-type massage apparatus 10 illustrated in Fig. 1. As illustrated in Fig. 1, the chair-type massage apparatus 10 has the seat section 11 on which the user is seated and a backrest section 12 that supports, from a back side, an upper body of the user who is seated on the seated section 11. Armrests 13 are provided at the left and right positions of the seat section 11, and a footrest 14 is provided in front of the seat section 11. The backrest section 12 is provided with a massage section (roller unit) 15. Although only the massage section 15 of the backrest section 12 is illustrated in the drawing, other parts also include massage sections such as air bags (omitted in the drawing).

In this embodiment, palpation can be performed by the aforementioned massage section 15. As the palpation, it is possible to press the back of the user along a route therein with the massage section 15, and muscular stiffness can be measured as will be described later. The muscular stiffness can be measured by pushing the massage section 15 against the user and measuring pressurizing force at that time with a pressure sensor.

In addition, a manipulator 20 for manipulating the massage apparatus 10 is provided at a part of the armrest 13 on the right side. The manipulator 20 according to this embodiment has a screen 21 that the user operates with a tablet-type manipulator, a sound input section (microphone) 22, and a sound output section (speaker) 23. Further, the massage apparatus 10 according to this embodiment includes a terminal device 40 in addition to the aforementioned manipulator 20. As the terminal device 40, a terminal device such as a smartphone, a tablet, or the like can be employed. A terminal device 40 that includes a sound input section (microphone) 22 and a sound output section (speaker) 23 can be utilized. The terminal device 40 can communicate with the massage apparatus 10 through wireless communication (Bluetooth (registered trademark)) 2. Note that the terminal device 40 may be connected to the massage apparatus 10 in another wireless or wired form.

In addition, the massage apparatus 10 according to the embodiment includes a body state acquisition section 30 that obtains a body state of the user. A blood pressure monitor 31 and a body composition analyzer 32 that are configured separately from the massage apparatus 10 are included as the body state acquisition section 30. The blood pressure monitor 31 and the body composition analyzer 32 can communicate with the massage apparatus 10 through the wireless communication (Bluetooth (registered trademark)) 2. The body state acquisition section 30 may be connected to the massage apparatus 10 in another wireless or wired form. In Addition, the body state acquisition section 30 may be incorporated in the massage apparatus 10. According to this example, it is possible to obtain, as the body state of the user, a "blood pressure" , "pulse rate" from the blood pressure monitor 31 and a "body weight" and a "body fat percentage" from the body composition analyzer. Note that a stress index measuring instrument or the like is included as the body state acquisition section 30. In A case in which the stress index measuring instrument is included, it is possible to obtain a "stress index" as a body state of the user. The body state of the user can be stored in a storage section 16, which will be described later. Note that an example in which the massage apparatus 10 is connected to a server 4 via the communication network 3 is illustrated in Fig. 1.

In addition, the massage apparatus 10 includes a control section 17 that controls the massage section 15, such as the seat section 11, the backrest section 12, the armrests 13, the footrest 14, and the like and an artificial intelligence section 50 that provides diagnosis in accordance with the user or appropriate advice based on data related to knowledge and know-how of doctors through a conversation with the user.

As illustrated in Fig. 2, the control section 17 is connected to the storage section 16. The control section 17 has, for example, a processor, a volatile memory, a non-volatile memory, an I/O interface, and the like. As the storage section 16, a flash memory, a hard disk drive, or the like can be used, for example. A plurality of massage courses with different content of massage provided by the aforementioned massage section 15 are stored in the storage section 16. The storage section 16 includes a function as an information storage section that stores individual information of the user. The individual information can include an "age", a "body weight", a "height", a "sex", a "family configuration", a "family medical history", and the like.

The control section 17 causes each massage section to operate in accordance with the plurality of massage courses or the like by the processor performing computation processing using the non-volatile memory on the basis of an operation program for each massage section stored in the storage section 16. The control section 17 can also change the content of the massage courses. In addition, it is also possible to cause only a part of content of massage as well as the massage courses to operate. The control section 17 performs control for strength of the operation performed by the massage section 15, a reclining angle of the backrest section 12, and the like.

Meanwhile, the artificial intelligence section 50 includes a computer or the like provided with artificial intelligence. The artificial intelligence section 50 has a function of understanding content of utterance from sound information of the user (the "sound information" in this specification and the claims includes "utterance information", and the "utterance information" includes "content of utterance"). The understanding of content of utterance by the artificial intelligence section 50 includes understanding of natural languages. The artificial intelligence section 50 has a learning function of accumulating information understood from the content of utterance in an information storage section 51 and utilizing the information for newly making determination.

As the data accumulated in the information storage section 51 of the artificial intelligence section 50, knowledge, know-how, and the like of doctors are accumulated. In addition, it is possible to accumulate knowledge and know-how of a plurality of doctors that are knowledge, know-how, and information of doctors from a variety of different fields (different fields of hospital departments, different fields of specialties in the same hospital department, and the like). In this manner, the artificial intelligence section 50 can consider a single symptom from different viewpoints on the basis of the knowledge and the know-how of the doctors from the variety of different fields, determine at least either diagnosis in accordance with the user or appropriate advice from diversified standpoints, and output either the diagnosis or the advice from the sound output section 23.

In addition, the artificial intelligence section 50 can accumulate the diagnosis or the appropriate advice provided to the user in the information storage section 51. By accumulating the diagnosis or the appropriate advice, it is possible to determine at least either new appropriate diagnosis or appropriate advice in accordance with the user at the time of reuse thereof in consideration of at least either previous diagnosis or appropriate advice accumulated in the information storage section 51, in addition to sound information of the user at the time of the reuse thereof. That is, the artificial intelligence section 50 can perform so-called follow-up diagnosis by comparing and examining the diagnosis and the appropriate advice accumulated in the information storage section 51 and the sound information of the user at the time of the reuse thereof. Then, it is possible to determine the diagnosis in accordance with the user or the appropriate advice at the time of the reuse thereof. Such a follow-up diagnosis function enables the user to reduce the time, the cost, and the effort to go to a hospital.

Further, the artificial intelligence section 50 can comprehensively determine diagnosis in accordance with the user and appropriate advice on the basis of the individual information stored (registered) in advance and the sound information input by the user. The artificial intelligence section 50 can provide the diagnosis and the advice in consideration of information of diseases in accordance with the "age", the "sex", and the like of the user. In addition, it is also possible to provide advice related to health in accordance with the age. In addition, it is possible to provide advice to the user on the basis of information such as a change in the "body weight" or the like. For example, the artificial intelligence section 50 can provide diagnosis of possible diseases and countermeasures therefore to the user or provide advice such as recommendation of an appropriate massage course in consideration of the individual information in addition to the sound information of the user. For example, it is possible to provide advice about a concern of a disease in accordance with symptoms, an appropriate hospital department, and the like on the basis of the sound information of the user and a family medical history.

In addition, results of state measurement such as a blood pressure value and a pulse rate from the blood pressure monitor 31 and a body weight and a body fat percentage from the body composition analyzer 32 and the like are sent as a body state of the user to the massage apparatus 10. Note that in a case in which a stress index measuring instrument is provided as the body state acquisition section 30, a result of measuring a state of a stress index is sent to the massage apparatus 10. Then, the artificial intelligence section 50 comprehensively determines diagnosis in accordance with the user or appropriate advice based on data related to the knowledge and the know-how of the doctors in accordance with the body state in addition to the sound information of the user and outputs the diagnosis or the advice from the sound output section 23. As the diagnosis in accordance with the user or the appropriate advice provided by the artificial intelligence section 50, it is possible to determine the diagnosis in accordance with the user or the appropriate advice from the data related to the knowledge and the know-how of the doctors stored in the information storage section 51 by extracting a plurality of keywords from content of a conversation with the user and content of utterance of the user and determining symptoms or the like of the user on the basis of the keywords. For example, it is possible to determine the diagnosis in accordance with the user or the appropriate advice from the data related to the knowledge and the know-how of the doctors in consideration of an autonomic nerve balance from an interval of a pulse (R-R interval) on the basis of a result of measurement of the body state of the user sent from the body state acquisition section 30.

Further, the aforementioned manipulator 20 according to the embodiment includes a camera 24 that serves as an imaging section in addition to the sound input section 22 and the sound output section 23. The camera 24 may be included in the terminal device 40. By including the camera 24, the artificial intelligence section 50 can provide diagnosis in accordance with the user and appropriate advice, such as recommendation of an appropriate massage course, suggestion of an exercise menu, suggestion of a food menu, or the like in consideration of information such as a skin color, skin firmness, and the like of the user captured by this camera 24 in addition to sound information of the user.

In addition, the massage apparatus 10 includes a clock section that has date and time information. The clock section can be included in the control section 17, for example. It is possible to determine diagnosis and appropriate advice in accordance with the use date and time of the user, for example, by including the clock section. If the date and the time when the user utilizes the massage apparatus 10 differ, the diagnosis in accordance with the user and the appropriate advice that the artificial intelligence section determines may differ even if the artificial intelligence section 50 obtains similar keywords from the sound information.

### Example of advice by massage apparatus

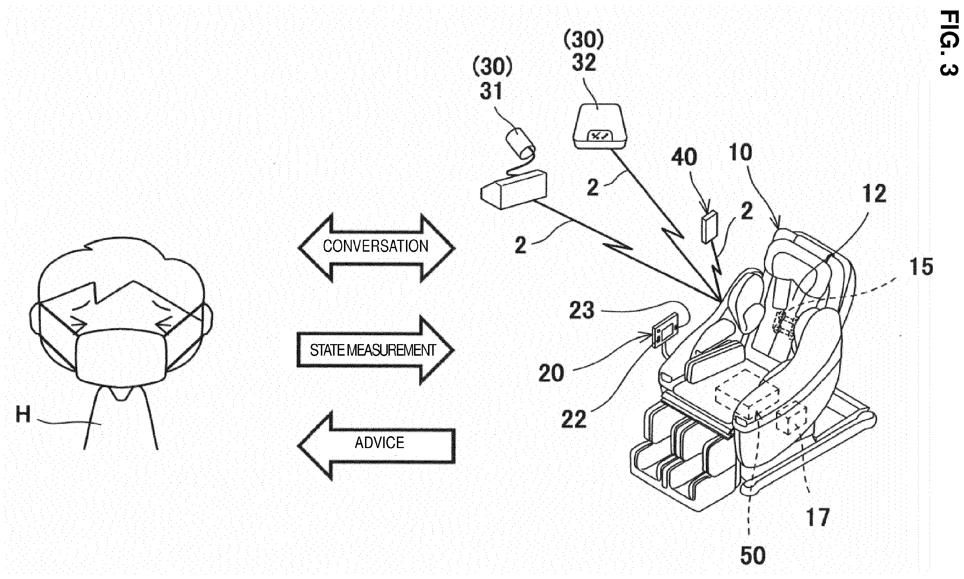

Fig. 3 is a diagram illustrating examples of a conversation between the massage apparatus 10 illustrated in Fig. 1 and a user H and advice. A basic conversation between the user H who utilizes the chair-type massage apparatus 10 and the artificial intelligence section 50 and advice will be described with reference to the drawing. Note that a configuration related to the massage apparatus 10 illustrated in Fig. 1 as described above will be described by applying the reference numerals thereto.

The user H make a conversation with the artificial intelligence section 50 of the massage apparatus 10. The conversation may be started from the user H or from the artificial intelligence section 50 in response to the user H turning on a power supply of the massage apparatus 10. If the user H talks toward the sound input section 22 of the massage apparatus 10 (input), the artificial intelligence section 50 understands content of the utterance from the sound information thereof. As for the understanding of the content of the utterance by the artificial intelligence section 50, it is possible to understand sound information of the user H (a feeling, a voice tone, a talking speed that can be determined from a way of speaking), utterance information (a site of interest and content related to massage), and the like. In the example in Fig. 1, results of state measurement such as a blood pressure value and a pulse rate from the blood pressure monitor 31 and a body weight, a body fat percentage, and the like from the body composition analyzer 32 are sent as a body state of the user H to the massage apparatus 10.

The user H can respond to the diagnosis or the advice by the artificial intelligence section 50 output from the sound output section 23 with sound from the sound input section 22. In this manner, it is possible to make a conversation with the artificial intelligence section 50 in relation to the diagnosis or the advice. That is, the user H can make a conversation with the artificial intelligence section 50 as if the user H talked to a doctor. The artificial intelligence section 50 can determine diagnosis further in accordance with the user H or further appropriate advice on the basis of new information obtained from the conversation and let the user H to know the diagnosis or the advice. For example, it is possible to encourage the user H to go to an appropriate hospital or to provide appropriate advice or to encourage the user H to receive massage for symptoms that are not so serious that the user H does not have to go to a hospital, in response to information (subjective symptoms or disease symptoms) that can be known from conversation with the user H by the artificial intelligence section 50.

According to the aforementioned massage apparatus 10, the user H can get diagnosis in accordance with the user H or appropriate advice regardless of a specialist on the basis of knowledge and know-how (information) of a plurality of doctors from a variety of fields at home in accordance with the symptoms of the user H through the conversation between the artificial intelligence section 50 and the user H and can examine countermeasures by the user themselves as described above. In a specific example in which the user H has a subjective symptom of a pain in stomach (for example, a pain in stomach due to minor stress), it is ordinarily possible to know that the factor of the symptom is stress by going to a hospital for diagnosis by MRI imaging, X-ray examination, or the like. In contrast, the artificial intelligence section 50 of the aforementioned massage apparatus 10 can determine that the factor of the pain in stomach is stress through the conversation with the user H. Then, the artificial intelligence section 50 can provide advice for solving the stress or recommend a massage course for solving the stress. Therefore, the user H can obtain information related to appropriate advice or effective massage at home for the symptoms that are not so serious that the user H does not have to go to a hospital.

In this manner, according to the aforementioned massage apparatus 10, the artificial intelligence section 50 can ask questions (medical interview) that are generally asked by doctors for diagnosis through the conversation between the user H and the artificial intelligence section 50. Then, it is possible to determine a disease name that corresponds to the site of the pain or the symptom of the user H and countermeasures for the disease on the basis of information (knowledge and know-how of a plurality of doctors) corresponding to diseases and symptoms in a variety of fields and affected parts and output the disease and the countermeasures by sound in response to the sound information of the respond. That is, it is possible to determine diagnosis in accordance with the user H or appropriate advice on the basis of knowledge and know-how of ten doctors from different hospital departments or from different specialized fields in the same hospital department, for example. Therefore, the user H can listen to the disease name and the countermeasures of the user H themselves, diagnosis, and appropriate advice from the massage apparatus 10 and can reduce a time, cost, and an effort to go to a hospital.

### Examples of factors on the basis of which diagnosis in accordance with user and appropriate advice is determined)

Hereinafter, examples of factors for the artificial intelligence section 50 to determine diagnosis in accordance with the user H or appropriate advice will be individually described.

### <Extraction of symptoms of user>

The artificial intelligence section 50 can extract symptoms of the user H at that time from sound information of the user H. As the symptoms of interest, symptoms such as a "fever", a "high blood pressure", "shoulder stiffness", "lower back pain", a "headache", a "stomachache", "constipation", "dizziness", "swelling", "unitary symptoms", "insomnia", "depressive symptoms", and the like can be extracted from content of utterance by the user H. For example, the artificial intelligence section 50 can extract information (keywords) such as "lower back hurts", "I have stiff shoulders", or the like from the sound information of the user H and determine symptoms of interest. In a case in which information that the user "continuously has a low-grade fever" from the content of the utterance by the user H, for example, it is possible to determine that the symptom relates to a "fever". In this manner, the artificial intelligence section 50 can extract the symptoms of interest of the user H at that time and determine the diagnosis in accordance with the user H or the appropriate advice based on the data related to the knowledge and know-how of the doctors for the symptoms.

Note that it is also possible to select symptom items of interest through the item selection section 25 in advance as will be described later. In this manner, since the extraction of symptoms by the artificial intelligence section 50 is omitted, it is possible to reduce the time for selecting the items through the determination made by the artificial intelligence section 50.

### <Extraction of information related to symptoms from questions and answers>

In a case in which the user H asks the artificial intelligence section 50 a question, the user H can make a question (utterance) such as "My legs are tired today", "My lower back hurts today", or "I have stiff shoulders today", for example. As a response from the artificial intelligence section 50 to the question "My lower back hurts today", for example, a further question such as "which part of the lower back hurts?", "Did you do deskwork all day long today?", or the like can be made for information related to symptoms in more detail.

Meanwhile, in a case in which the artificial intelligence section 50 asks the user H a question, the artificial intelligence section 50 can make a question such as "Do you have any part where you have felt a pain recently?", "Do you have any stiff part?", or the like. Then, in a case in which a response such as "I have a pain in my lower back" is obtained from the user H, the artificial intelligence section 50 can repeat a question such as "which part of your lower back hurts?" for information related to symptoms in more detail.

In this manner, questions and answers can be repeated between the user H and the artificial intelligence section 50. The artificial intelligence section 50 can continue a conversation to obtain new responses by asking questions related to responses from the user H. The artificial intelligence section 50 can repeat questions to induce content of the responses from the user H in order to get information to determine the symptoms of the user H in detail. Then, the artificial intelligence section 50 can determine symptoms from the plurality of responses obtained from the user H and determine diagnosis in accordance with the user H, appropriate advice, and appropriate massage on the basis of data related to the knowledge and the know-how of the doctors for the symptoms.

### <Extraction of activity information>

The artificial intelligence section 50 can determine activity information of the user H from the sound information of the user H. For example, it is possible to determine activity information about how the user H has spent the day from the content of the utterance. For example, activity information such as "the user did standing work all day long", "the user worked outside all day long", "the user did deskwork all day long", or the like can be extracted. Then, the artificial intelligence section 50 can determine the diagnosis in accordance with the user H or the appropriate advice in consideration of the activity information in addition to the sound information of the user H. In a case in which the user H did deskwork all day long, for example, it is possible to determine that the lower back and the back are tired and to provide diagnosis and advice from the symptoms occurring from the deskwork. In addition, it is possible to recommend massage mainly for the lower back and the back or a massage course for stimulating acupuncture points that are effective for fatigue of the lower back and the back.

### <Diagnosis based on visual examination>

The artificial intelligence section 50 can determine a feeling and a health state from a face expression and a face color from imaging information of the face of the user H captured by the camera 24 on the basis of visual examination. In this manner, the artificial intelligence section 50 can determine diagnosis in accordance with the user H, advice, an appropriate massage course, or the like in consideration of a result of the determination by the visual examination in addition to the sound information of the user H.

### <Utilization of use history>

The artificial intelligence section 50 can store the diagnosis in accordance with the user H or the appropriate advice in the information storage section 51. Then, the artificial intelligence section 50 can determine the diagnosis in accordance with the user H or the appropriate advice at the time of reuse thereof in consideration of diagnosis or appropriate advice in the past that is stored in the information storage section 51 in addition to new sound information of the user H. For example, it is possible to determine diagnosis in accordance with the user H or the appropriate advice on the day by comparing and examining symptoms and the diagnosis or the appropriate advice of the user H on the previous day with symptoms of the use H extracted from the sound information on that day. By storing the diagnosis or the appropriate advice for the user H in this manner, it is possible to include consideration of the diagnosis or the advice in the past in a conversation or a determination made by the artificial intelligence section 50 at the time of the reuse by the user H.

### <Other Considerations>

The artificial intelligence section 50 can determine the diagnosis in accordance with the user H or the appropriate advice in consideration of information such as a season, epidemic diseases, and the like obtained from a clock section in addition to sound information of the user H. For example, it is possible to determine the diagnosis in accordance with the user H or the appropriate advice based on the data related to the knowledge and the know-how of doctors in consideration of seasonal information such as epidemic period of diseases such as "influenza", a period with a large temperature difference due to a change in seasons, and the like as information related to the use date and time (a time zone, a season, and the like) of the user H in addition to sound information of the user H.

### <Combinations>

It is possible to combine the aforementioned factors on the basis of which the artificial intelligence section 50 determines the diagnosis in accordance with the user H or the appropriate advice, the factors are appropriately combined by the artificial intelligence section 50 in accordance with conditions or the like. In addition, the factors on the basis of which the artificial intelligence section 50 determines the diagnosis in accordance with the user H or the appropriate advice as described above are just examines, it is possible to add various factors without departing from the gist of the present invention, and the factors are not limited to the aforementioned examples.

### Example of advice provided by massage apparatus

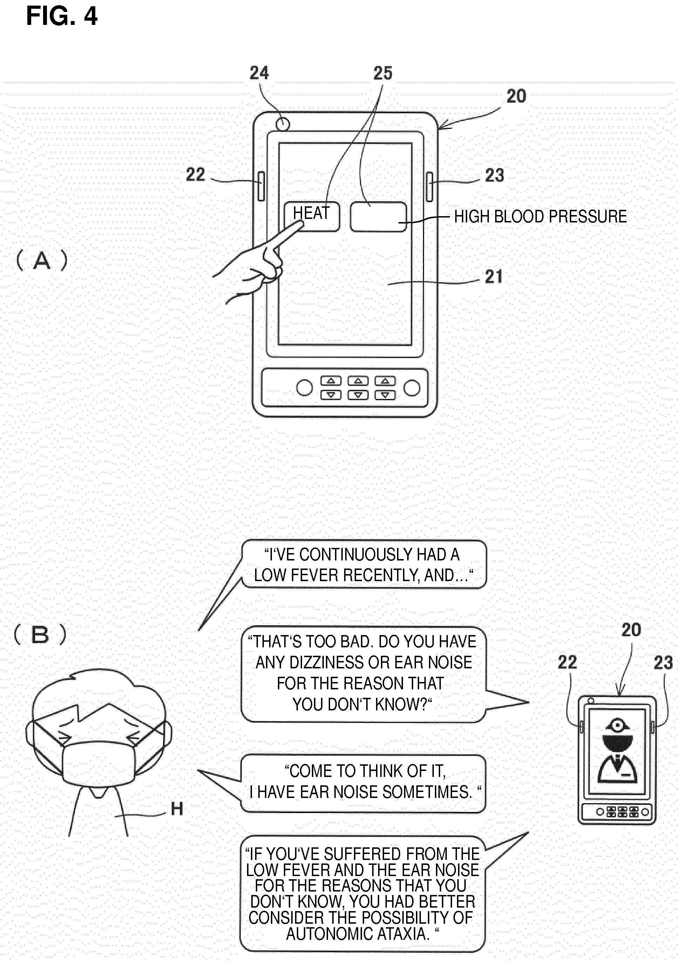

Fig. 4 is a diagram illustrating an example of a conversation between the massage apparatus 10 illustrated in Fig. 1 and the user H and an example of advice, (A) is a diagram illustrating an example of item selection, and (B) is a diagram illustrating a specific example of a conversation. An example of a conversation between the massage apparatus 10 and the user H and advice will be described with reference to these drawings. This example is an example of diagnosis or advice provided by the artificial intelligence section 50 in relation to a "fever" of the user H. Note that the configuration illustrated in Fig. 1 will be described by applying the same reference numerals.

An example case in which the user H thinks "I've continuously had a low fever recently, and I'm worried about it though I caught a cold according to the previous diagnosis at the hospital..." and "I want to get a second opinion, but it's not easy to take a time to go to the hospital again, and also, extra money is needed...", for example, will be described.

In such a case, according to the aforementioned massage apparatus 10, the user H inputs sound information such as "I've continuously had a low fever recently, and..." to the sound input section 22 after pressing the "fever" in the item selection section 25. In this case, since the artificial intelligence section 50 can recognize that the symptoms relate to the "fever", the artificial intelligence section 50 asks a question such as "That's too bad. Do you have any dizziness or ear noise for the reason that you don't know?". Then, if the user H answers that "Come to think of it, I have ear noise sometimes", the artificial intelligence section 50 outputs diagnosis in accordance with the symptoms of the user H such as "If you've suffered from the low fever and the ear noise for the reasons that you don't know, you had better consider the possibility of autonomic ataxia" from the sound output section 23.

As described above, the user H can obtain diagnosis in accordance with the symptoms of the user H themselves from the massage apparatus 10. Although the simple example has been described above, the artificial intelligence section 50 can determine diagnosis or appropriate advice in accordance with symptoms from diversified standpoints on the basis of knowledge and know-how of a plurality of doctors from different hospital departments or from different specialized fields such that the user H can obtain a plurality of diagnosis results or appropriate advice matters.

### Example of advice provided by massage apparatus

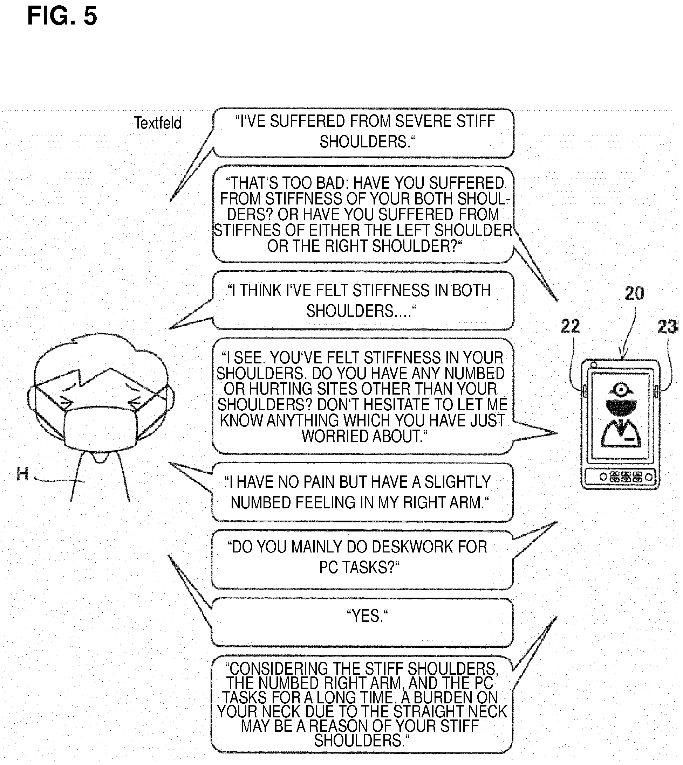

Fig. 5 is a diagram illustrating a different specific example of a conversation between the massage apparatus 10 illustrated in Fig. 4 and the user H. This example is an example of diagnosis or advice provided by the artificial intelligence section 50 in relation to "shoulder stiffness" of the user H. Note that the configuration illustrated in Fig. 1 will be described by applying the same reference numerals thereto.

For example, if the user H inputs sound information such as "I've suffered from severe stiff shoulders", the artificial intelligence section 50 asks questions such as "That's too bad. Have you suffered from stiffness of your both shoulders?", "Or have you suffered from stiffness of either the left shoulder or the right shoulder?" Then, if the user H answers that "I think I've felt stiffness in both shoulders...", the artificial intelligence section 50 asks questions such as "I see. You've felt stiffness in your shoulders. Do you have any numbed or hurting sites other than your shoulders?", "Don't hesitate to let me know anything which you have just worried about." Then, if the user H answers that "I have no pain but have a slightly numbed feeling in my right arm", the artificial intelligence section 50 asks a question such as "Do you mainly do deskwork for PC tasks?" Then, if the user H answers "Yes", the artificial intelligence section 50 outputs diagnosis in accordance with the symptoms of the user H such as "Considering the stiff shoulders, the numbed right arm, and the PC tasks for a long time, a burden on your neck due to the straight neck may be a reason of your stiff shoulders" from the sound output section 23.

In this manner, the user H can obtain diagnosis in accordance with the symptoms of the user H of themselves from the massage apparatus 10. Although the simple example has been described above, the artificial intelligence section 50 can determine diagnosis or appropriate advice in accordance with symptoms from diversified standpoints on the basis of knowledge and know-how of a plurality of doctors from different hospital departments or from different specialized fields such that the user H can obtain a plurality of diagnosis results or appropriate advice matters.

### Example of palpation provided by massage apparatus

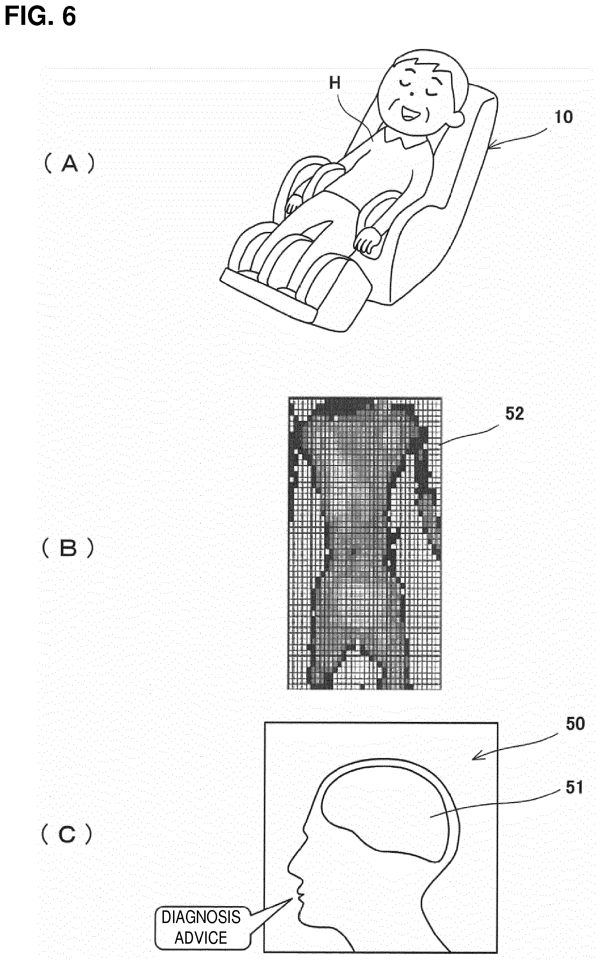

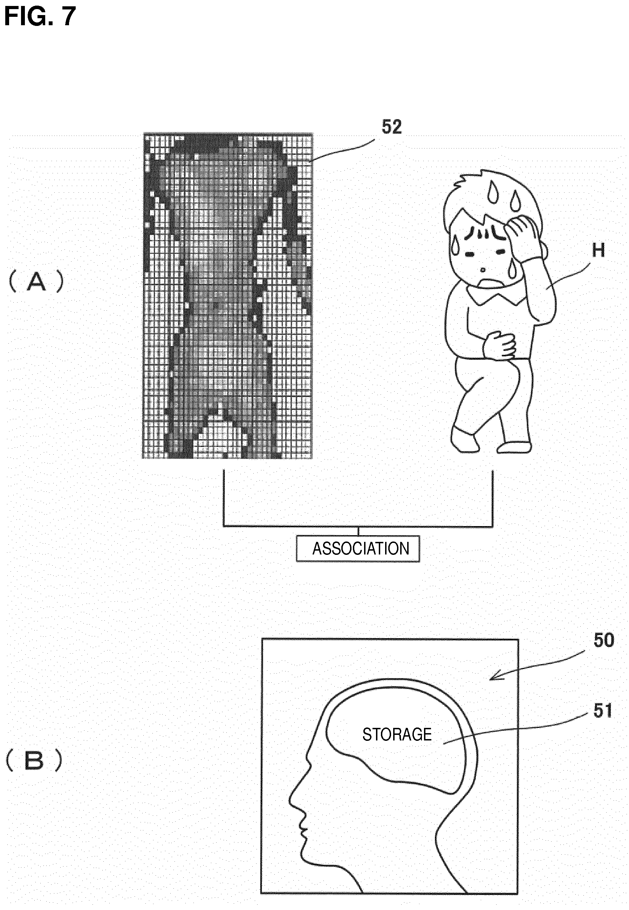

Figs. 6(A) to 6(C) are diagrams illustrating relationship of palpation by the massage apparatus illustrated in Fig. 1, an example of a mapping image of muscular stiffness, and the artificial intelligence section. Figs. 7(A) and 7(B) are diagrams illustrating an example of accumulated data in which the example of the mapping image of the muscular stiffness illustrated in Fig. 6 and a health state of the user H are associated. Note that Figs. 6(C) and 7(B) are diagrams schematically illustrating the artificial intelligence section 50 and the information storage section 51.

As illustrated in Fig. 6(A), according to the aforementioned chair-type massage apparatus 10, it is possible to measure muscular stiffness by pressing the massage section 15 (Fig. 1) against the back of the user H along the route of the back and measuring pressurizing force at that time with the pressure sensor.

As illustrated in Fig. 6(B), the muscular stiffness obtained by the aforementioned palpation is mapped by the artificial intelligence section 50, and a result of the mapping can be represented as a mapping image 52. The user H can view the mapping image 52 on the screen 21 (Fig. 1) and know the muscular stiffness of the user H themselves.

Then, as illustrated in Fig. 6(C), the artificial intelligence section 50 can provide diagnosis in accordance with the user H and appropriate advice in consideration of the mapping image 52 of the muscular stiffness in addition to sound information of the user H. For example, if only the right shoulder is stiff from hardness of muscle at each part, an overall balance, and the like, it is possible to predict a liver disease, a gastrointestinal disorder, or the like. If only the left shoulder is stiff, it is possible to predict a heart disease or the like. If the root of the neck is stiff or hurts, it is possible to predict subarachnoid hemorrhage or the like. In this manner, it is possible not only to determine a current health state but also to predict a health state in the future from the mapping image 52 of muscular stiffness. Further, the artificial intelligence section 50 can consider imaging information, such as a skin color, skin firmness, and the like, of the user H captured by the aforementioned camera 24 in addition to the muscular stiffness. For example, it is possible to determine the health state of the user H as described above and to determine diagnosis in accordance with the user H or appropriate advice based on data related to knowledge and know-how of doctors from the mapping image of the muscular stiffness.

The mapping image 52 obtained by the aforementioned palpation can be associated (linked) with the health state of the user H As illustrated in Fig. 7(A) and can be stored in the information storage section 51 of the artificial intelligence section 50 as illustrated in Fig.7(B). In this manner, it is possible to determine diagnosis in accordance with the user H at that time and appropriate advice by periodically measuring muscular stiffness and taking a temporal change in mapping images 52 thereof into consideration.

### Other embodiments

As represented by the two-dotted chain line in Fig. 1, the control section 17 or the artificial intelligence section 50 of the aforementioned massage apparatus 10 may be connected to the server 4 via the communication network 3. In this manner, it is possible to update data related to knowledge and know-how of doctors accumulated in the information storage section 51 of the artificial intelligence section 50 to the latest state via the communication network 3. In addition, it is also possible to increase the amount of data by adding new information via the communication network 3. Further, in a case in which the server 4 includes big data, it is also possible to determine diagnosis in accordance with the user H or appropriate advice in further detail by the artificial intelligence section 50 utilizing the big data in the server 4. In addition, the artificial intelligence section 50 can provide advice such as a hospital located near the user H from the information obtained from the communication network 3 on the basis of result of diagnosis in accordance with the user H or appropriate advice.

If the massage apparatus 10 is a massage system 1 connected to the communication network 3 in this manner, the artificial intelligence section 50 can determine diagnosis in accordance with the user H or appropriate advice on the basis of sound information of the user H at the time of utilization from the latest data related to knowledge and know-how of doctors.

### Overview

As described above, the aforementioned massage apparatus 10 can determine diagnosis in accordance with the user H or appropriate advice based on data related to knowledge and know-how of doctors in accordance with symptoms such as a health state of the user H at that time from conversation between the user H and the artificial intelligence section 50 and to let the user H to know the diagnosis or the advice. In addition, the user H can know a recommended massage course that is appropriate for body and mind states when the user H uses the chair-type massage apparatus 10, the fact that massage is preferably inhibited, and the like. Further, it is possible to obtain diagnosis in accordance with the user H or appropriate advice based on knowledge and know-how of a plurality of doctors from a plurality of different fields and to obtain diagnosis or advice for various symptoms from the massage apparatus 10.

Therefore, the user H can know, from the massage apparatus 10, diagnosis in accordance with the user H themselves, appropriate advice, an appropriate massage course, or the like in accordance with a physical condition, a feeling, or the like that changes day by day.

### Other modification examples

Although the chair-type massage apparatus 10 has been described as an example in the aforementioned embodiments, the massage apparatus 10 may be a massage apparatus of a type other than the chair type, may be a lower back massage apparatus, a neck massage apparatus, or the like as long as the massage apparatus massages the user H, and the massage apparatus is not limited to that of the chair-type.

In addition, the aforementioned embodiments illustrate examples, content of the conversation between the user H and the artificial intelligence section 50 can be different due to various factors, various conversations can be made without departing from the gist of the present invention, and the present invention is not limited to the aforementioned embodiments.

### LIST OF REFERENCE NUMERALS

- 1: massage system
- 2: wireless communication
- 3: communication network
- 4: server
- 10: massage apparatus (chair-type massage apparatus)
- 12: backrest section
- 15: massage section (roller unit)
- 16: storage section
- 17: control section (clock section)
- 20: manipulator
- 21: screen
- 22: sound input section
- 23: sound output section
- 24: camera (imaging section)
- 30: body state acquisition section
- 31: blood pressure monitor (body state acquisition section)
- 32: body composition analyzer (body state acquisition section)
- 40: terminal device
- 50: artificial intelligence section
- 51: information storage section
- 52: mapping image
- H: user

## Claims

1. A massage apparatus that has a massage section for massaging a user, the apparatus comprising:
a sound input section;
a sound output section; and
an artificial intelligence section that determines diagnosis and advice based on data accumulated at least in relation to knowledge and know-how of doctors,
wherein the artificial intelligence section is configured to determine at least either of diagnosis in accordance with the user or appropriate advice based on the data at least in relation to the knowledge and the know-how of the doctors, on the basis of sound information of the user input from the sound input section through a conversation with the user and outputs at least either the diagnosis or the advice from the sound output section.

2. The massage apparatus according to claim 1,
wherein the artificial intelligence section is configured to determine at least either the diagnosis in accordance with the user or the appropriate advice based on the data accumulated at least in relation to knowledge and know-how of a plurality of doctors in a plurality of different fields.

3. The massage apparatus according to claim 1 or 2, further comprising:
an item selection section through which the user provides inputs,
wherein the artificial intelligence section is configured to determine at least either the diagnosis in accordance with the user or the appropriate advice based on the data accumulated in relation to the knowledge and know-how of the doctors, on the basis of the sound information of the user, in relation to an item selected by the user through the item selection section.

4. The massage apparatus according to any one of claims 1 to 3, further comprising:
a body state acquisition section that acquires a body state of the user,
wherein the artificial intelligence section is configured to determine at least either the diagnosis in accordance with the user or the appropriate advice in consideration of the body state obtained by the body state acquisition section in addition to the sound information of the user.

5. The massage apparatus according to any one of claims 1 to 4, further comprising:
a storage section that stores individual information of the user,
wherein the artificial intelligence section is configured to determine at least either the diagnosis in accordance with the user or the appropriate advice in consideration of the individual information saved in the storage section in addition to the sound information of the user.

6. The massage apparatus according to any one of claims 1 to 5, comprising:
an imaging section that images the user,
wherein the artificial intelligence section is configured to determine at least either the diagnosis in accordance with the user or the appropriate advice in consideration of imaging information captured by the imaging section in addition to the sound information of the user.

7. The massage apparatus according to any one of claims 1 to 6,
wherein the artificial intelligence section is configured to accumulate at least either the diagnosis or the appropriate advice for the user in the information storage section and determine at least either new diagnosis in accordance with the user at the time of reuse thereof or appropriate advice in consideration of at least either the diagnosis or the appropriate advice accumulated in the information storage section in addition to sound information of the user at the time of the reuse thereof.

8. The massage apparatus according to any one of claims 1 to 7, comprising:
a clock section that has at least date and time information,
wherein the artificial intelligence section is configured to determine at least either the diagnosis in accordance with the user or the appropriate advice in consideration of use date and time information of the user obtained from the clock section.

9. The massage apparatus according to any one of claims 1 to 8,
wherein the massage apparatus is a chair-type massage apparatus that has at least a seat section on which the user is seated, a backrest section that supports, from a back side, an upper body of the user who is seated on the seat section, the massage section that performs massage, and a control section that controls the massage section, and
the artificial intelligence section is configured to conduct palpation with the massage section on the user who is seated on the seat section and determine at least either the diagnosis in accordance with the user and the appropriate advice in consideration of a result of the palpation in addition to the sound information of the user.

10. The massage apparatus according to any one of claims 1 to 9,
wherein the artificial intelligence section is configured to recommend an appropriate massage course or encourage inhibition of the massage on the basis of a result of determining at least either the diagnosis or the appropriate advice.

11. A massage system comprising:
the massage apparatus according to any one of claims 1 to 10,
wherein the artificial intelligence section is connected to a server via a communication network and is configured to be able to update the accumulated data related to the knowledge and the know-how of the doctors.
